# EUROPEAN PATENT SPECIFICATION

(11) **EP 2 571 563 B1**
(45) Date of publication and mention of the grant of the patent: **13.10.2021**
(21) Application number: 11784194.0
(22) Date of filing: 18.05.2011
(51) Int. Cl.: A61M 37/00, A61M 39/02, A61M 39/00

(54) **REINFORCED SEPTUM FOR AN IMPLANTABLE MEDICAL DEVICE**
VERSTÄRKTES SEPTUM FÜR EINE IMPLANTIERBARE MEDIZINISCHE VORRICHTUNG
CLOISON RENFORCÉE POUR DISPOSITIF MÉDICAL IMPLANTABLE

(30) Priority: 18.05.2010 US 345846 P
(43) Date of publication of application: 27.03.2013
(60) Divisional application: 21192999.7
(73) Proprietor: C.R. Bard, Inc., Murray Hill, NJ 07974 (US)
(72) Inventor: HIBDON, Dwight, T., Park City UT 84098 (US); CHRISTIAN, Kelly, J., Draper UT 84020 (US); FARNWORTH, Charles, Riverton UT 84065 (US)
(74) Representative: Hoffmann Eitle
(86) International application number: PCT/US2011/037038
(87) International publication number: WO 2011/146649

(56) References cited:
- WO-A1-99/34859
- GB-A- 2 191 701
- US-A- 4 857 053
- US-A- 6 039 712
- US-A1- 2003 208 184
- US-A1- 2006 264 898
- US-A1- 2009 227 964
- US-A1- 2009 227 964

## Description

### CROSS-REFERENCE TO RELATED APPLICATIONS

GB-2.191.701 discloses a septum comprising a needle penetrable seal member wherein the seal member is locked in an opening of a needle stop member by a clamp ring member. A wall extension of the needle stop member is rolled over onto the clamping ring.

### BRIEF SUMMARY

Briefly summarized, embodiments of the present invention are directed to a septum for use in sealably covering a fluid cavity of an implantable medical device, such as an access port. The needle-penetrable septum is resilient and includes a reinforcement structure that bolsters septum placement over the fluid cavity so as to inhibit unintended separation of the septum from the medical device when the fluid cavity is under pressure, such as during power injection of fluid into the fluid cavity. The reinforcement structure further assists in preventing septum separation during other septum stress events including insertion of a needle through the septum to access the port fluid cavity, or removal of the needle from the septum when access is no longer needed.

In one embodiment the septum comprises a resilient septum body that includes a flange disposed about a perimeter thereof. A reinforcement component is disposed in the flange for reinforcing engagement of the flange with a corresponding groove defined about an opening to the fluid cavity of the medical device so as to inhibit unintended detachment of the septum from the medical device. The reinforcement component in one embodiment includes an annular cord disposed in the flange.

Various methods for inserting the reinforced septum into the groove in order to sealably cover the fluid cavity are also disclosed, including temporary deformation of the septum, oversizing of the outer diameter of the groove, and the use of memory shape materials for the reinforcement cord. A septum configured as described herein can thus be secured to the access port without the need of an additional cap or retaining ring. This in turn enables an integral access port body of single-piece construction to be employed, which provides simplified design and construction.

These and other features of embodiments of the present invention will become more fully apparent from the following description and appended claims, or may be learned by the practice of embodiments of the invention as set forth hereinafter.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 is a perspective view of an access port that serves as one possible environment in which an embodiment of the present disclosure can be practiced;
FIG. 1A is a cross sectional side view of a portion of an implantable access port including a septum configured in accordance with one embodiment;
FIGS. 2A and 2B respectively show top and cross sectional side views of a septum configured in accordance with one embodiment;
FIGS. 3A and 3B respectively show perspective and cross sectional side views of an access port body configured in accordance with one embodiment;
FIGS. 4A and 4B respectively show perspective and cross sectional side views of an access port in accordance with one embodiment;
FIG. 5 is a cross sectional side view of an access port body in accordance with one embodiment;
FIG. 6 is cross sectional side view of an access port including a septum according to one embodiment;
FIG. 7 is a cross sectional side view of an access port including a septum according to another embodiment;
FIG. 8A-8D are cross sectional side views of possible access port groove shapes in accordance with one embodiment;
FIGS. 9A-9C are cross sectional side views showing insertion of a septum into a groove of an access port;
FIG. 10 is a top view of a septum including a segmented cord in accordance with one embodiment; and
FIG. 11 is a cross sectional side view of a portion of an implantable access port including a septum configured in accordance with one embodiment.

### DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a transcutaneous catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

Embodiments of the present invention are generally directed to a septum for use in sealably covering a reservoir, or fluid cavity, of an implantable medical device, such as an access port. The septum is needle-penetrable so as to enable piercing thereof by a needle or other suitable cannula or device to infuse fluids into, or remove fluids from, the fluid cavity.

In one embodiment, the septum is resilient and includes a reinforcement structure that bolsters septum placement over the fluid cavity so as to prevent blow-out of the septum, *i.e.*, unintended separation of the septum from the medical device when the fluid cavity is under pressure, such as during pressurized injection of fluid into the fluid cavity of an access port. Power injection of fluids into an access port is one example of such pressurized fluid injection, wherein fluid is injected into the fluid cavity of the access port at a rate of about five ml per second at a pressure of about 300 psi, for example. Other fluid pressure scenarios are also possible.

In one embodiment, the reinforcement structure of the septum includes an annular bead, or cord, disposed within a flange circumventing an outer perimeter of the septum. The cord is configured to provide for reinforced retention of the septum flange within a groove defined about an opening of the fluid cavity of the access port such that the septum sealably covers the fluid cavity. So configured, the septum is secured to the access port without the need of an additional cap or retaining ring. Indeed, in one embodiment an integral access port body of single-piece construction can be used, thus providing simplified design and construction.

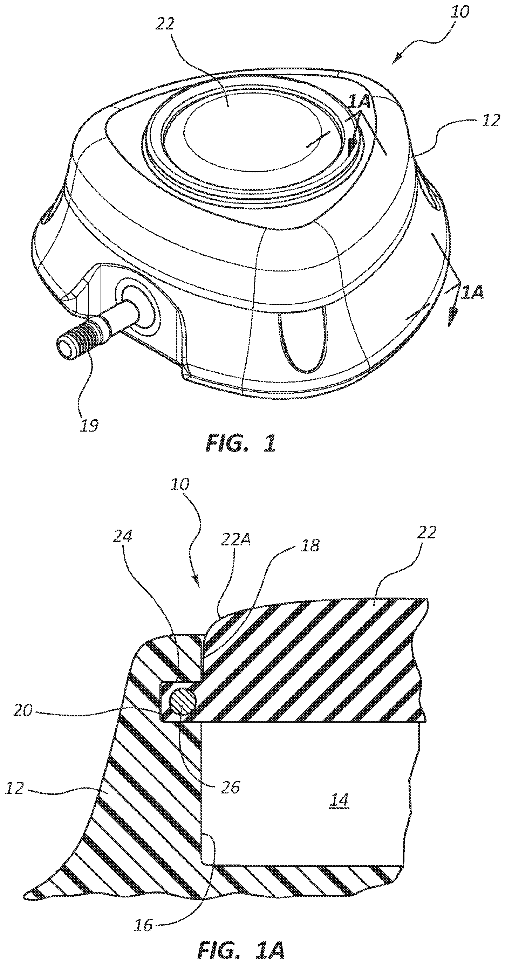

Reference is first made to FIGS. 1 and 1A, which depict various details regarding an implantable access port, generally designated at 10, according to one embodiment. The port 10 includes a body 12 including a suitable material. In one embodiment, the port body 12 includes plastic, such as an acetyl resin for instance, though it is appreciated that other suitable plastics and materials can be employed for the port body, including titanium or other metals or combinations of metals, plastics, or both. The body 12 defines a generally cylindrical fluid cavity 14 (though other cavity shapes are possible) defined in part by a wall 16. A stem 19 in fluid communication with the fluid cavity 14 is also included so that fluids can be passed to and from the fluid cavity.

As best seen in FIG. 1A, the body 12 defines an opening 18 to the fluid cavity 14. An annular recess, or groove 20, is defined in the wall 16 proximate the fluid cavity opening 18. The groove 20 generally defines a rectangular cross sectional shape and further defines an outer diameter ("OD") 20A. Note that other cross sectional shapes are possible for the groove. The groove 20 is sized to receive therein a flange of a septum, described below.

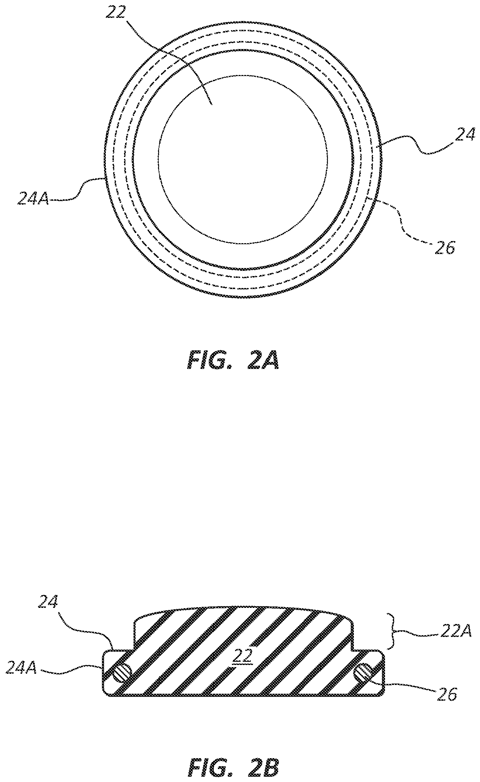

As mentioned, and with additional reference to FIGS. 2A and 2B, the access port 10 further includes a septum 22 disposed in the opening 18 and configured to sealably cover the fluid cavity 14. The septum 22 includes a suitable material such as silicone that enables it to be penetrated by a needle or other cannula so as to provide fluid access to the fluid cavity 14, such as for the infusion into or removal of fluids therefrom. The silicone or other suitable material included in the septum 22 also enables the septum to be resiliently compliant.

As best seen in FIGS. 2A and 2B, the septum 22 in the present embodiment is circular and includes an annular flange 24 extending about a perimeter of the septum. The flange 24 itself defines an outer diameter ("OD") 24A and is sized and configured to be received within the groove 20 such that the septum 22 is disposed in the opening 18 and sealably covers the fluid cavity 14. So disposed, the septum 22 further defines an upper portion 22A disposed above the flange 24, as viewed from the perspective shown in FIG. 1A.

As seen in FIGS. 1A, 2A, and 2B, a reinforcement structure is included in the septum 22. In particular, an annular reinforcement bead, or cord 26, is included within the septum flange 24. Though shown fully encapsulated by the septum flange 24, the reinforcement cord 26 in one embodiment could be partially exposed. It is appreciated that the reinforcement cord 26 can be included within the septum flange 24 as shown in FIGS. 1A, 2A, and 2B via any one of a plurality of methods, including insert molding of the cord into the septum, mechanical insertion into the flange after cutting a circular slit therein followed by re-sealing of the slit, etc. Also, the reinforcement cord shown here includes a round cross sectional profile; it is appreciated, however, that the cord can include other cross sectional profiles, including square, triangular, pentagonal, polygonal, oval, twisted, slotted, spring coiled, etc.

The reinforcement cord 26 is non-resilient along its circular axis such that, when the flange 24 is received within the groove 20 of the fluid cavity 14, the cord provides rigidity to the flange and prevents deformation thereof. This in turn prevents blow-out or unintended detachment of the septum 22 from the port body 12, especially when the port fluid cavity 14 is under pressurization, such as during power injection, for instance. So configured, then, the reinforcement cord reinforces, or bolsters, engagement of the flange with the groove of the port body.

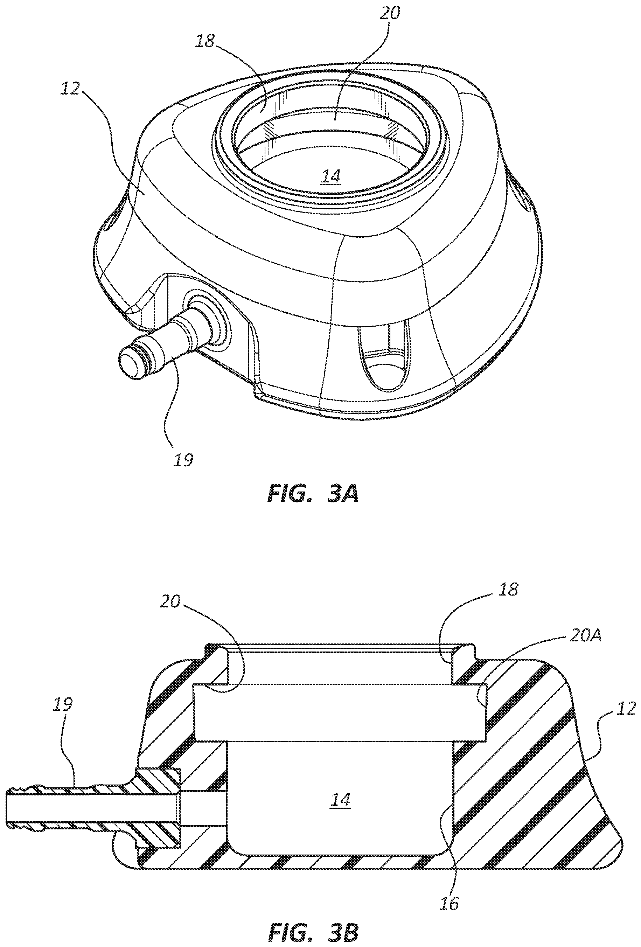

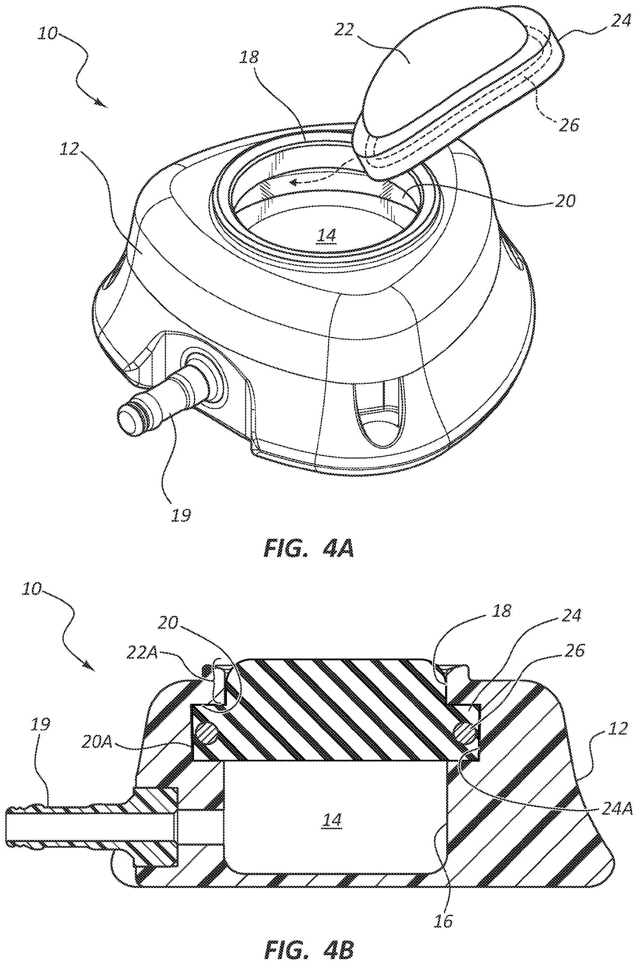

FIGS. 3A and 3B show details of the port body 10 before attachment of the septum 22 thereto. In one embodiment, placement of the septum 22 in the access port 10 can include deforming the septum, such as by partially folding in the manner shown in FIG. 4A. Note that even though it is substantially non-elastic in a direction parallel to its circular axis, in one embodiment the reinforcement cord is capable of a limited amount of deformation in other directions, such as the folding deformation shown in FIG. 4A.

Once partially folded as in FIG. 4A, the septum 22 can then be inserted into the fluid cavity 14 via the opening 18 such that a portion of the reinforced septum flange 24 seats within the groove 20. Portions of the flange 24 adjacent the seated portion can then be worked into the groove 20 until the flange is completely seated therein and the septum 22 is no longer deformed, as in FIG. 4B. In one embodiment, it is appreciated that full seating of the septum flange occurs with the assistance of temporary pressurization and/or depressurization of the fluid cavity.

In another embodiment, the septum can be placed in the fluid cavity via the assistance of a placement tool to temporarily deform the septum so as to insert its periphery into the port body groove. In one embodiment, the tool includes a tube that radially compresses the septum and a plunger that pushes the compressed septum out the end of the tube and into the groove of the port body. In another embodiment, the tool includes a funnel shaped member in which the septum is initially disposed, and further including a rolling rod that can be pressed along the periphery of the septum to pass the septum periphery out of the funnel and into the port body groove. These and other insertion tools are therefore contemplated.

In one embodiment, the reinforcement cord 26 includes a plastic, such as polypropylene, acetyl resin, or other plastic or thermoplastic that includes suitable stiffness so as to prevent buckling under hoop stress. In other embodiments, the cord can include any suitable material such as woven or non-woven metals, plastics, ceramics, high durometer silicones, titanium, stainless steel, nylons, copolymers, and acrylics, etc. In yet another embodiment, the reinforcement cord can include a silicone with a durometer rating that renders it harder relative to a hardness of the septum itself.

In one embodiment the reinforcement cord includes a nickel and titanium alloy commonly known as nitinol, which is a shape memory material. So configured, the reinforcement cord can be initially formed and disposed within the septum while defining a first shape configuration that facilitates relatively simple placement of at least a portion of the septum flange into the groove of the port fluid cavity. Such a first shape configuration of the cord can cause a first half of the septum to assume a partial folded shape while a second half of the septum remains unfolded, for instance. After the unfolded portion of the septum is seated in the groove. the septum can be heated so as to activate the shape memory characteristic of the nitinol reinforcement cord, which causes the cord to change shape to a predetermined second shape configuration. In one embodiment, the second shape configuration is a circularly flat cord configuration, such as that shown in FIGS. 2A and 2B, for instance. Shaping of the reinforcement cord to the second shape configuration causes the formerly folded half of the septum to flatten, which enables the rest of the septum flange to seat into the groove and sealably cover the fluid cavity. Thus, the septum disposed in a final flat configuration with the entire flange is disposed within the groove.

As mentioned, once the septum flange is fully seated within the groove, the reinforcement cord acts as a strengthening element to inhibit unintended removal of the septum from its position covering the fluid cavity of the implantable access port. In addition, in one embodiment the reinforcement cord maintains the septum flange in place within the groove so as to enable construction of the access port body as an integral, single-piece component, *i*.*e*., without the need for a retaining ring or cap to retain the septum in place. This in turn simplifies construction and design of the access port.

Note that, though an access port is shown and described herein, in other embodiments other implantable medical devices that include a septum secured thereto can also benefit from the principles of the present disclosure. As such, the present discussion should not be intended to limit the embodiments of the present disclosure in any way.

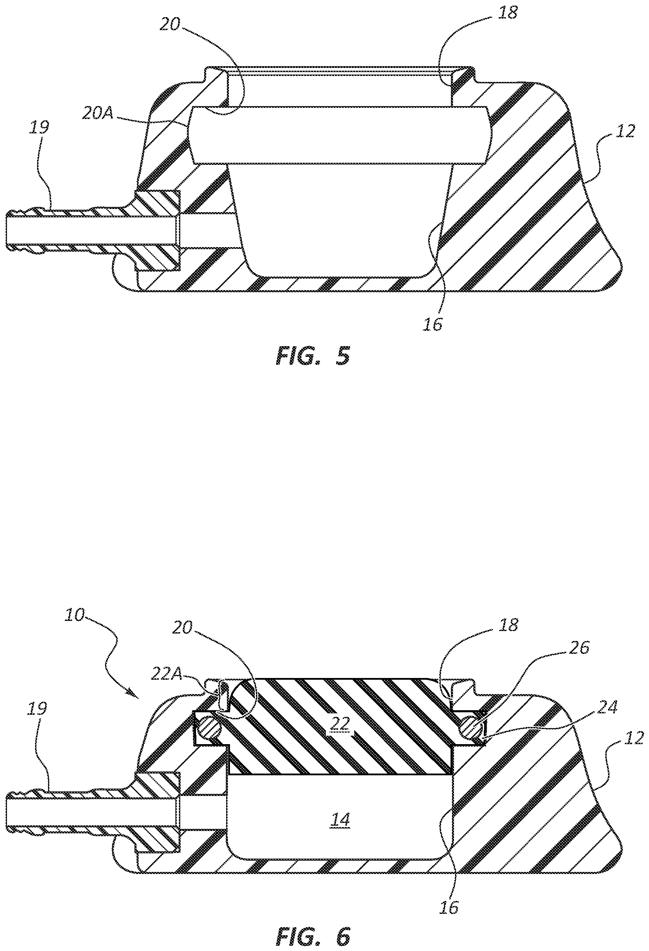

FIG. 5 shows that in one embodiment, the fluid cavity can be shaped other than cylindrically. For example the side wall 16 of the port body 12 in FIG. 5 is slanted so as to define a frustoconically shaped fluid cavity 14. This configuration can be employed to assist in urging the septum flange 24 to seat in the groove 20. Other fluid cavity shapes are also possible. More generally, it is appreciated that the top view cross sectional shapes of the septum and fluid cavity can also vary from circular, including oval shapes for instance.

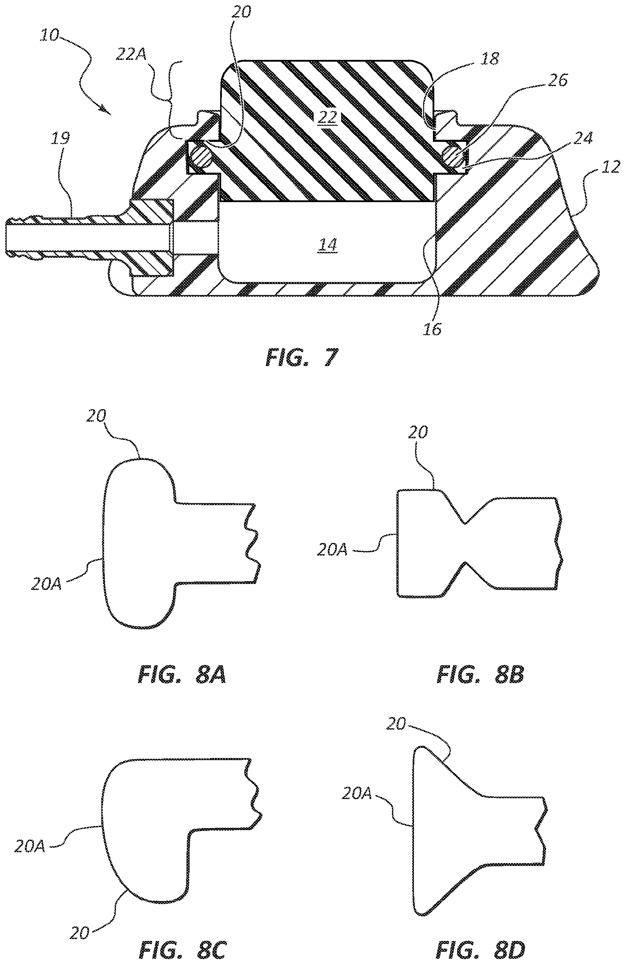

As mentioned further above, note that the cross sectional shape of the groove can vary from the rectangular configuration shown in FIG. 1A. For instance, in one embodiment the OD 20A of the groove 20 can define a concave shape. In another embodiment, the groove can include a convex, or cupped, shape as shown in FIG. 5 so as to assist in retaining the septum flange. In one embodiment, the OD 24A of the flange 24 can be correspondingly shaped to match the groove OD 20A. Further non-limiting examples of possible cross sectional shapes for the groove 20 are shown in FIGS. 8A-8D.

It is appreciated that the position of the groove in the fluid cavity can vary according to design. Moreover, the thickness of the groove and corresponding septum flange can also vary from what is shown and described herein. These and other variations are therefore contemplated.

FIGS. 6 and 7 show that the size of the septum 22 can vary from thin (FIG. 6), including a relatively thin septum upper portion 22A to thick (FIG. 7), wherein the septum upper portion is relatively thick. In the case of the thin septum 22 of FIG. 6, an area of radial compression includes a majority of the thickness of the septum, while the thick septum of FIG. 7 includes only a lower portion of the septum thickness where radial compression is present. Note that the cord-equipped septum discussed herein enables a septum to be mated to the access port body without unduly increasing the thickness of the port, thus preserving a low profile port configuration, if desired.

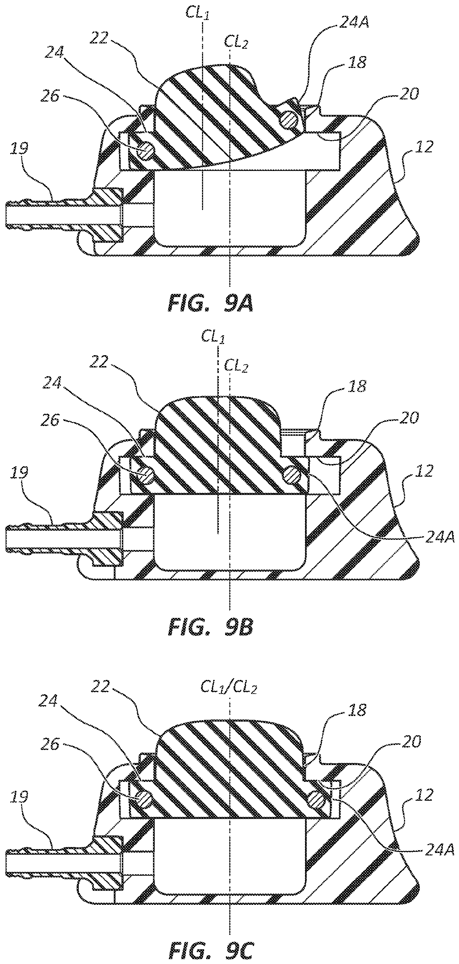

FIGS. 9A-9C show that, in one embodiment, the OD 20A of the groove 20 can be greater relative to the OD 24A of the septum flange 24 such that ease of insertion of the septum flange into the groove is facilitated. In particular, these figures show a process for inserting the septum into the port body including laterally compressing the septum 22 such that its centerline CL₁ is offset from the centerline CL₂ of the port body 12 while inserting a portion of the septum flange 24 into the groove 20. With the septum still laterally compressed, the opposing portion of the flange 24 is inserted into the groove 20. Such insertion is made possible due to the oversized nature of the groove OD 20A. Once the flange 24 has been seated in the groove 20, compression of the septum 22 is released, and the septum centers in the opening 18 such that is centerline CL₁ substantially aligns with the centerline CL₂ of the port body 12.

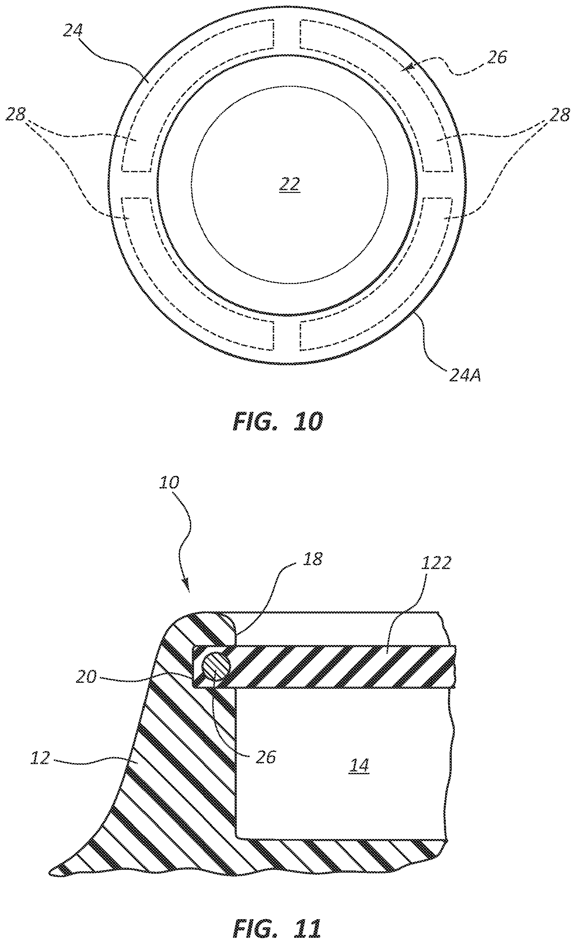

FIG. 10 shows that in one embodiment the reinforcement cord can include other than a continuous configuration. Indeed, FIG. 10 shows the reinforcement cord as including a plurality of reinforcement segments 28 that are arranged in a generally circular pattern and disposed within the flange 24 of the septum 22. These and other reinforcement schemes are therefore contemplated. In another embodiment, a plurality of reinforcement cords can be included in the septum flange. FIG. 11 shows that, in one embodiment, a septum 122 that includes no upper portion can also include the reinforcement cord 26, thus illustrating the ability to include a reinforcement cord in septa of varying types and configurations.

The described embodiments are to be considered in all respects only as illustrative, not restrictive. The scope of the embodiments is, therefore, indicated by the appended claims rather than by the foregoing description. All changes that come within the meaning and range of equivalency of the claims are to be embraced within their scope.

## Claims

1. A septum (22; 122) for sealably covering a fluid cavity (14) defined in an implantable device, comprising:
a resilient septum body; and
a reinforcement component **characterised in that** the reinforcement component is disposed in the septum body proximate a periphery thereof for reinforcing engagement of the periphery with a corresponding groove (20) defined about an opening (18) to the fluid cavity of the medical device so as to inhibit unintended detachment of the septum from the medical device, enabling employment of an integral access port body of single piece construction.

2. The septum (22; 122) as defined in claim 1, wherein the perimeter of the septum is circular, wherein the septum defines at the periphery thereof an annular flange (24), and wherein the reinforcement component includes at least one reinforcement cord (26) disposed within the flange of the septum, wherein preferably the flange includes a rectangular cross sectional shape.

3. The septum (22; 122) as defined in claim 2, wherein the reinforcement cord includes a plurality of discrete segments (28).

4. The septum (22; 122) as defined in claim 2, wherein the reinforcement cord is a continuous annular cord including a circular cross sectional shape, and wherein the reinforcement cord is non-resilient along an annular axis thereof.

5. The septum (22; 122) as defined in any of claims 1-4, wherein the reinforcement cord includes a memory shape material and is capable of assuming a first shape configuration during placement of the septum flange in the groove and a second shape configuration after at least partial placement of the flange in the groove.

6. The septum (22; 122) as defined in any of claims 2-5, wherein the septum includes silicone, and wherein the reinforcement cord is insert-molded within the flange of the septum, wherein preferably the reinforcement cord includes at least one of polypropylene, acetyl resin, silicone, titanium, and stainless steel.

7. An implantable access port (10), comprising:
a body (12) defining a fluid cavity (14) and a recess (20); and a septum (22; 122) defined in any of claims 1-6.

8. The access port (10) defined in claim 7, wherein the septum is a needle-penetrable septum covering the fluid cavity, the septum including:
a flange (24) that is received within the recess (20) of the port body; and
a reinforcement component disposed at least partially within the flange,
the reinforcement structure bolstering engagement of the flange with the recess.

9. The access port (10) defined in claim 8, wherein the port includes at least one of a thermoplastic such as acetyl resin and a metal, wherein the recess includes an annular groove (20) defined proximate an opening of the fluid cavity, and wherein the flange is annularly defined about the septum, wherein preferably an outer diameter of the groove is greater relative to an outer diameter of the flange so as to enable a first portion of the septum flange to be seated within the groove followed by a second opposing portion of the septum flange to be seated within the groove.

10. The access port (10) as defined in claim 8 or 9, wherein the reinforcement component includes an annular cord (26) disposed within the flange of the septum so as to prevent removal of the flange from the recess when the access port is subjected to power injection.

11. The access port as defined in claim 9, wherein the access port includes a single-piece body that defines the fluid cavity and the annular groove, and wherein the flange is inserted into the groove by first deforming the septum.

12. A method for assembling an implantable medical device, the method comprising:
providing an implantable medical device defining a fluid cavity (14) with an opening (18) and a groove (20);
providing a septum (22; 122) as defined in any of the claims 1-6;
placing the septum in a first shape configuration;
inserting at least a portion of the periphery into the groove; and placing the septum in a second shape configuration such that a remainder of the periphery is seated within the groove and the septum covers the fluid cavity of the medical device,
wherein preferably placing the septum in the first shape configuration is accomplished via assistance from an insertion tool.

13. The method for assembling as defined in claim 12, wherein the reinforcement cord includes a shape memory material, wherein placing the septum in the first shape configuration includes forming the reinforcement cord such that the cord constrains the septum into a shape that facilitates insertion of at least a portion of the flange into the groove, and wherein placing the septum in a second shape configuration includes activating the shape memory of the reinforcement cord such that the cord assumes a final configuration with the entire flange seated within the groove.

14. The method for assembling as defined in claim 12, wherein placing the septum in the first shape configuration includes deforming at least a portion of the septum so as to enable passage of a portion of the septum through the opening, and wherein placing the septum in the second shape configuration includes allowing the septum to return to a natural, untwisted state.

15. The method for assembling as defined in claim 12, wherein the groove is an annular groove (24) disposed proximate the opening of the fluid cavity, wherein the flange of the septum is annular, wherein an outer diameter of the groove is larger relative to an outer diameter of the annular flange, wherein placing the septum in the first shape configuration includes pushing a centerline (CL₁) of the septum past a centerline (CL₂) of fluid cavity of the port while inserting a portion of the flange into the groove, and wherein placing the septum in the second shape configuration includes releasing the septum so that the remainder of the flange seats within the groove and the centerlines of the septum and the fluid cavity align.

## Patentansprüche

1. Septum (22; 122) zum abgedichteten Abdecken eines Fluidhohlraums (14), der in einer implantierbaren Vorrichtung definiert ist, umfassend:
einen elastischen Septumkörper; und
eine Verstärkungskomponente, **dadurch gekennzeichnet, dass** die Verstärkungskomponente in dem Septumkörper in der Nähe zu einer Peripherie davon angeordnet ist für verstärkendes Eingreifen der Peripherie in eine entsprechende Rille (20), die um eine Öffnung (18) zu dem Fluidhohlraum der medizinischen Vorrichtung so definiert ist, dass unbeabsichtigtes Lösen des Septums von der medizinischen Vorrichtung unterbunden wird, wodurch der Einsatz eines integrierten Zugangsanschlusskörpers als einteilige Konstruktion ermöglicht wird.

2. Septum (22; 122) nach Anspruch 1, wobei der Umfang des Septums kreisförmig ist, wobei das Septum an seiner Peripherie einen ringförmigen Flansch (24) definiert, und wobei die Verstärkungskomponente mindestens einen Verstärkungsstrang (26) einschließt, der innerhalb des Flanschs des Septums angeordnet ist, wobei der Flansch vorzugsweise eine rechteckige Querschnittsform einschließt.

3. Septum (22; 122) nach Anspruch 2, wobei der Verstärkungsstrang eine Vielzahl von diskreten Segmenten (28) einschließt.

4. Septum (22; 122) nach Anspruch 2, wobei der Verstärkungsstrang ein durchgehender ringförmiger Strang ist, der eine kreisförmige Querschnittsform einschließt, und wobei der Verstärkungsstrang entlang einer ringförmigen Achse davon nicht elastisch ist.

5. Septum (22; 122) nach einem der Ansprüche 1 ― 4, wobei der Verstärkungsstrang ein Formgedächtnis-Material einschließt und fähig ist, eine erste Formkonfiguration während des Einsetzens des Septumflanschs in die Rille und eine zweite Formkonfiguration nach mindestens teilweisem Einsetzen des Flanschs in die Rille anzunehmen.

6. Septum (22; 122) nach einem der Ansprüche 2 - 5, wobei das Septum Silikon einschließt, und wobei der Verstärkungsstrang innerhalb des Flanschs des Septums einsatzgeformt ist, wobei der Verstärkungsstrang vorzugsweise mindestens eines von Polypropylen, Acetylharz, Silikon, Titanium und Edelstahl einschließt.

7. Implantierbarer Zugangsanschluss (10), umfassend:
einen Körper (12), der einen Fluidhohlraum (14) und eine Aussparung (20) definiert; und ein Septum (22; 122) nach einem der Ansprüche 1-6.

8. Zugangsanschluss (10) nach Anspruch 7, wobei das Septum ein mit einer Nadel durchstoßbares Septum ist, das den Fluidhohlraum abdeckt, wobei das Septum Folgendes einschließt:
einen Flansch (24), der innerhalb der Aussparung (20) des Anschlusskörpers aufgenommen ist; und
eine Verstärkungskomponente, die mindestens teilweise innerhalb des Flanschs angeordnet ist, wobei die Verstärkungsstruktur Eingreifen des Flanschs in die Aussparung untermauert.

9. Zugangsanschluss (10) nach Anspruch 8, wobei der Anschluss mindestens eines von einem Thermoplast, wie Acetylharz, und einem Metall einschließt, wobei die Aussparung eine ringförmige Rille (20) einschließt, die in der Nähe einer Öffnung des Fluidhohlraums definiert ist, und wobei der Flansch ringförmig um das Septum definiert ist, wobei vorzugsweise ein Außendurchmesser der Rille größer ist als ein Außendurchmesser des Flanschs, um es so zu ermöglichen, dass ein erster Abschnitt des Septumflanschs innerhalb der Rille abgesetzt wird, gefolgt von einem zweiten gegenüberliegenden Abschnitt des Septumflanschs, der innerhalb der Rille abgesetzt wird.

10. Zugangsanschluss (10) nach Anspruch 8 oder 9, wobei die Verstärkungskomponente einen ringförmigen Strang (26) einschließt, der innerhalb des Flanschs des Septums so angeordnet ist, dass Entfernen des Flanschs aus der Aussparung verhindert wird, wenn der Zugangsanschluss Injektion unter Kraft unterzogen wird.

11. Zugangsanschluss nach Anspruch 9, wobei der Zugangsanschluss einen einteiligen Körper einschließt, der den Fluidhohlraum und die ringförmige Rille definiert, und wobei der Flansch durch Verformen des Septums in die Rille eingesetzt wird.

12. Verfahren zum Zusammenbau einer implantierbaren medizinischen Vorrichtung, wobei das Verfahren Folgendes umfasst:
Bereitstellen einer implantierbaren medizinischen Vorrichtung, die einen Fluidhohlraum (14) mit einer Öffnung (18) und einer Rille (20) definiert;
Bereitstellen eines Septums (22; 122) nach einem der Ansprüche 1 ― 6;
Versetzen des Septums in eine erste Formkonfiguration;
Einsetzen von mindestens einem Abschnitt der Peripherie in die Rille; und Versetzen des Septums in eine zweite Formkonfiguration, so dass ein Rest der Peripherie innerhalb der Rille abgesetzt wird und das Septum den Fluidhohlraum der medizinischen Vorrichtung abdeckt, wobei Versetzen des Septums in die erste Formkonfiguration vorzugsweise über Zutun eines Einsetzwerkzeugs erreicht wird.

13. Verfahren zum Zusammenbau nach Anspruch 12, wobei der Verstärkungsstrang ein Formgedächtnis-Material einschließt, wobei Versetzen des Septums in die erste Formkonfiguration Formen des Verstärkungsstrangs einschließt, so dass der Strang das Septum in eine Form zwängt, die den Einsatz von mindestens einem Abschnitt des Flanschs in die Rille erleichtert, und wobei Versetzen des Septums in eine zweite Formkonfiguration Aktivieren des Formgedächtnisses des Verstärkungsstrangs einschließt, so dass der Strang eine endgültige Konfiguration mit dem gesamten Flansch innerhalb der Rille abgesetzt einnimmt.

14. Verfahren zum Zusammenbau nach Anspruch 12, wobei Versetzen des Septums in die erste Formkonfiguration Verformen von mindestens einem Abschnitt des Septums so einschließt, um den Durchgang eines Abschnitts des Septums durch die Öffnung zu ermöglichen, und wobei Versetzen des Septums in die zweite Formkonfiguration Zulassen einschließt, dass das Septum in einen natürlichen, nicht verdrehten Zustand zurückkehrt.

15. Verfahren zum Zusammenbau nach Anspruch 12, wobei die Rille eine ringförmige Rille (24) ist, die in der Nähe der Öffnung des Fluidhohlraums angeordnet ist, wobei der Flansch des Septums ringförmig ist, wobei ein Außendurchmesser der Rille größer ist als ein Außendurchmesser des ringförmigen Flanschs, wobei Versetzen des Septums in die erste Formkonfiguration Drücken einer Mittellinie (CL₁) des Septums über eine Mittellinie (CL₂) des Fluidhohlraums des Anschlusses hinaus während des Einsetzens eines Abschnitts des Flanschs in die Rille einschließt, und wobei Versetzen des Septums in die zweite Formkonfiguration Freigeben des Septum einschließt, so dass der Rest des Flanschs innerhalb der Rille sitzt und sich die Mittellinien des Septums und des Fluidhohlraums ausrichten.

## Revendications

1. Cloison (22; 122) pour recouvrir de manière étanche une cavité de fluide (14) définie dans un dispositif implantable, comprenant :
un corps de cloison élastique ; et
un composant de renforcement, **caractérisé en ce que** le composant de renforcement est disposé dans le corps de cloison à proximité d'une périphérie de celui-ci pour renforcer une mise en prise de la périphérie avec une rainure correspondante (20) définie autour d'une ouverture (18) vers la cavité de fluide du dispositif médical de manière à empêcher un détachement involontaire de la cloison à partir du dispositif médical, permettant une utilisation d'un corps d'orifice d'accès intégral de construction d'une seule pièce.

2. Cloison (22; 122) selon la revendication 1, dans laquelle le périmètre de la cloison est circulaire, dans laquelle la cloison définit à sa périphérie une bride annulaire (24), et dans laquelle le composant de renforcement inclut au moins un cordon de renforcement (26) disposé à l'intérieur de la bride de la cloison, dans laquelle de préférence la bride inclut une forme de section transversale rectangulaire.

3. Cloison (22; 122) selon la revendication 2, dans laquelle le cordon de renforcement inclut une pluralité de segments distincts (28).

4. Cloison (22; 122) selon la revendication 2, dans laquelle le cordon de renforcement est un cordon annulaire continu incluant une forme de section transversale circulaire, et dans laquelle le cordon de renforcement n'est pas élastique le long d'un axe annulaire de celui-ci.

5. Cloison (22; 122) selon l'une quelconque des revendications 1-4, dans laquelle le cordon de renforcement inclut un matériau à mémoire de forme et est capable de prendre une première configuration de forme pendant un placement de la bride de cloison dans la rainure et une seconde configuration de forme après un placement au moins partiel de la bride dans la rainure.

6. Cloison (22 ; 122) selon l'une quelconque des revendications 2-5, dans laquelle la cloison inclut de la silicone, et dans laquelle le cordon de renforcement est moulé par insertion à l'intérieur de la bride de la cloison, dans laquelle de préférence le cordon de renforcement inclut au moins un parmi du polypropylène, une résine acétyle, de la silicone, du titane et de l'acier inoxydable.

7. Orifice d'accès implantable (10), comprenant :
un corps (12) définissant une cavité de fluide (14) et un évidement (20) ; et une cloison (22; 122) définie dans l'une quelconque des revendications 1-6.

8. Orifice d'accès (10) selon la revendication 7, dans lequel la cloison est une cloison pouvant être pénétrée par une aiguille recouvrant la cavité de fluide, la cloison incluant :
une bride (24) qui est reçue à l'intérieur de l'évidement (20) du corps d'orifice; et un composant de renforcement disposé au moins partiellement à l'intérieur de la bride, la structure de renforcement amplifiant une mise en prise de la bride avec l'évidement.

9. Orifice d'accès (10) selon la revendication 8, dans lequel l'orifice inclut au moins un élément parmi un matériau thermoplastique tel qu'une résine acétyle et un métal, dans lequel l'évidement inclut une rainure annulaire (20) définie à proximité d'une ouverture de la cavité de fluide, et dans lequel la bride est définie de manière annulaire autour de la cloison, dans lequel de préférence un diamètre extérieur de la rainure est supérieur à un diamètre extérieur de la bride de manière à permettre à une première partie de la bride de cloison d'être logée à l'intérieur de la rainure puis à une seconde partie opposée de la bride de cloison d'être logée à l'intérieur de la rainure.

10. Orifice d'accès (10) selon la revendication 8 ou 9, dans lequel le composant de renforcement inclut un cordon annulaire (26) disposé à l'intérieur de la bride de la cloison de manière à empêcher un retrait de la bride à partir de l'évidement lorsque l'orifice d'accès est soumis à une injection d'énergie.

11. Orifice d'accès selon la revendication 9, dans lequel l'orifice d'accès inclut un corps d'une seule pièce qui définit la cavité de fluide et la rainure annulaire, et dans lequel la bride est insérée dans la rainure tout d'abord en déformant la cloison.

12. Procédé d'assemblage d'un dispositif médical implantable, le procédé comprenant les étapes consistant à :
fournir un dispositif médical implantable définissant une cavité de fluide (14) avec une ouverture (18) et une rainure (20) ;
fournir une cloison (22; 122) telle que définie dans l'une quelconque des revendications 1-6 ;
placer la cloison dans une première configuration de forme ;
insérer au moins une partie de la périphérie dans la rainure ; et placer la cloison dans une seconde configuration de forme de telle sorte qu'un reste de la périphérie soit logé dans la rainure et la cloison recouvre la cavité de fluide du dispositif médical, dans lequel de préférence le placement de la cloison dans la première configuration de forme est réalisé à l'aide d'un outil d'insertion.

13. Procédé d'assemblage selon la revendication 12, dans lequel le cordon de renforcement inclut un matériau à mémoire de forme, dans lequel le placement de la cloison dans la première configuration de forme inclut une formation du cordon de renforcement de telle sorte que le cordon contraint la cloison dans une forme qui facilite une insertion d'au moins une partie de la bride dans la rainure, et dans lequel un placement de la cloison dans une seconde configuration de forme inclut une activation de la mémoire de forme du cordon de renforcement de telle sorte que le cordon prenne une configuration finale avec la bride entière logée à l'intérieur de la rainure.

14. Procédé d'assemblage selon la revendication 12, dans lequel le placement de la cloison dans la première configuration de forme inclut une déformation d'au moins une partie de la cloison de manière à permettre un passage d'une partie de la cloison à travers l'ouverture, et dans lequel le placement de la cloison dans la seconde configuration de forme inclut une autorisation de la cloison à revenir à un état naturel non tordu.

15. Procédé d'assemblage selon la revendication 12, dans lequel la rainure est une rainure annulaire (24) disposée à proximité de l'ouverture de la cavité de fluide, dans lequel la bride de la cloison est annulaire, dans lequel un diamètre extérieur de la rainure est plus grand par rapport à un diamètre extérieur de la bride annulaire, dans lequel le placement de la cloison dans la première configuration de forme inclut une poussée d'une ligne centrale (CL₁) de la cloison au-delà d'une ligne centrale (CL₂) de cavité de fluide de l'orifice tout en insérant une partie de la bride dans la rainure, et dans lequel le placement de la cloison dans la seconde configuration de forme inclut une libération de la cloison de sorte que le reste de la bride se place à l'intérieur de la rainure et les lignes centrales de la cloison et de la cavité de fluide s'alignent.
